# EUROPEAN PATENT SPECIFICATION

(11) **EP 2 202 217 B1**
(45) Date of publication and mention of the grant of the patent: **26.06.2013**
(21) Application number: 09168209.6
(22) Date of filing: 09.07.2002
(51) Int. Cl.: C07C 209/36, C07C 209/68, C07C 211/56

(54) **Process for preparing 4-aminodiphenylamines**
Verfahren zur Herstellung von 4-Aminodiphenylaminen
Procédé de préparation de 4-aminodiphénylamines

(30) Priority: 23.07.2001 US 911058; 10.05.2002 US 143478
(43) Date of publication of application: 30.06.2010
(62) Divisional of application: 02742396.1
(73) Proprietor: FLEXSYS AMERICA L.P., Akron, OH 44333-0444 (US)
(72) Inventor: Triplett II, Ralph, Dale, Wadsworth, OH 44281 (US); Rains, Roger, Keranen, Richfield, Ohio 44286 (US)
(74) Representative: Beetz, Tom

(56) References cited:
- EP-A- 0 566 783
- WO-A-00/35853
- US-A- 5 117 063
- US-A- 5 453 541
- US-A- 5 608 111
- US-A- 5 739 403
- YURI GOLDBERG ET AL: "betaines derived from amino and hydrazino acids as phase transfer catalysts" TETRAHEDRON, ELSEVIER SCIENCE PUBLISHERS, AMSTERDAM, NL LNKD- DOI:10.1016/S0040-4020(01)89759-3, vol. 46, no. 6, 1 January 1990 (1990-01-01), pages 1911-1922, XP002113890 ISSN: 0040-4020
- STERN, M.K. ET AL.: "Direct coupling of aniline and nitrobenzene: A new example of nucleophilic aromatic substitution for hydrogen" J. AM. CHEM. SOC., vol. 114, no. 23, 1992, pages 9237-9238, XP002216864

## Description

### BACKGROUND OF THE INVENTION

### 1. Field of the Invention

The present invention relates to a process for preparing 4-aminodiphenylamines intermediates.

### 2. Related Art

4-Aminodiphenylamines are widely used as intermediates in the manufacture of alkylated derivatives having utility as antiozonants and antioxidants, as stabilizers for monomers and polymers, and in various specialty applications. For example, reductive alkylation of 4-aminodiphenylamine (4-ADPA) with methylisobutyl ketone provides N-(1,3-dimethylbutyl)-N'-phenyl-p-phenylene-diamine, which is a useful antiozonant for the protection of various rubber products.

4-Aminodiphenylamine derivatives can be prepared in various ways. An attractive synthesis is the reaction of an optionally substituted aniline with an optionally substituted nitrobenzene in the presence of a base, as disclosed, for example, in U.S. 5,608,111 (to Stern et al.) and U.S. 5,739,403 (to Reinartz et al.).

U.S. 5,608,111 describes a process for the preparation of an optionally substituted 4-ADPA wherein in a first step optionally substituted aniline and optionally substituted nitrobenzene are reacted (coupled) in the presence of a base. In working examples, aniline and nitrobenzene are reacted in the presence of tetramethylammonium hydroxide as the base, and water and aniline are azeotropically removed during the coupling reaction.

International publication WO 00/35853 discloses a method of preparation of intermediates of 4-aminodiphenylamine by the reaction of aniline with nitrobenzene in a liquid medium where the reaction system consists of a solution of salts of true zwitterions with hydroxides. A combination of potassium hydroxide and betaine hydrate is exemplified. The reaction may take place in the presence of free oxygen.

EP publication 566 783 describes a method of manufacture of 4-nitrodiphenylamine by the reaction of nitrobenzene with aniline in the medium of a polar aprotic solvent in a strongly alkaline reaction system. A phase transfer catalyst such as tetrabutylammonium hydrogen sulfate is employed. This reference requires that the reaction be carried out in an oxygen-free atmosphere in order to prevent undesirable side reactions caused by oxidation.

US Patent No. 5,117,063 and International publication WO 01/14312 disclose processes for preparing 4-nitrodiphenylamine and 4-nitrosodiphenylamine, using an inorganic base with crown ether, a phase transfer catalyst.

US Patent No. 5,453,541 teaches that an external desiccant, such as anhydrous sodium sulfate, may be used to absorb excess water in an anaerobic or aerobic process for producing one or more 4-ADPA intermediates in which substituted aniline derivatives and nitrobenzene are brought into reactive contact.

The objective of the present invention is to provide a superior method for producing one or more 4-ADPA intermediates by reacting aniline and nitrobenzene in the presence of a strong base and a phase transfer catalyst, or in the presence of an organic base and an inorganic salt or a metal organic salt.

### SUMMARY OF THE INVENTION

In brief summary, in one embodiment, the present invention is for a method of producing 4-aminodiphenylamine comprising the steps of:
(a) bringing an aniline derivative selected from the group consulting of formanilide, phenylurea, carbamilide and thiocarbamilide derivative into reactive contact with nitrobenzene;
(b) obtaining a 4-aminodiphenylamine intermediate product by reacting the aniline derivative and nitrobenzene in a confined zone at a suitable time, pressure and temperature, in the presence of a mixture comprising a strong base having pk₆ less than 9.4 an oxidant and a phase transfer catatyst wherein the phase transfer catalyst is selected from the group consisting of tetramethylammonium chloride, tetramethylammonium fluoride, tetramethylammonium hydroxide, bis-tetramethylammonium carbonate, tetramethylammonium formate and tetramethylammonium acetate; tetrabutylammonium hydrogensulfate, tetrabutylammonium sulfate; methyltributylammonium chloride; benzyltrimethylammonium hydroxide, tricaprylmethylammonium chloride, tetrabutylammonium chloride, tetramethylammonium nitrate, cetyltrimethylammonium chloride and choline hydroxide;
(c) reducing the 4-aminodiphenylamine intermediate product of step (b) to produce 4-aminodiphenylamine; and
(d) optionally, wherein the 4-aminodiphenylamine produced in reductively allylated to an alkylated derivative of the 4-aminodiphenyl-amine.

Other embodiments of the present invention encompass details about reaction mixtures and ratios of ingredients, particular phase transfer catalysts and particular strong bases, all of which are hereinafter disclosed in the following discussion of the present invention.

### DETAILED DESCRIPTION OF THE INVENTION

The present invention is directed to a method, as described above, for making intermediates of 4-ADPA that has superior yield and selectivity for those intermediates. Such intermediates comprise 4-nitroso- and/or 4-nitrodiphenylamines (*p*-NDPA and 4-NDPA, respectively) and salts thereof. The intermediates may then be hydrogenated to produce 4-aminodiphenylamine.

An example of a substituted and multifunctional organic base is (2S, 3S)-bis(trimethylammonio)-1,4-butanediol dihydroxide. Other effective organic bases in addition to those shown in the following examples, can be derived from the above phase transfer catalysts, wherein the anion is replaced by hydroxide or other suitable anion form.

Phase transfer catalysts of the present invention have several advantages over crown ethers, such as 18-crown-6, which were described as effective with alkali metal hydroxides in references such as US Patent No. 5,117,063 and International publication WO 01/14312 discussed above. The most obvious disadvantages of crown ethers are very high initial cost and high toxicity. In addition, most crown ethers have poor solubility in water, so they cannot be recovered for recycle with an aqueous base stream. Furthermore, the boiling points of crown ethers are high enough that they cannot be recovered by distillation without an extra distillation step. Even for the class of crown ethers that have good solubility in water, solubility in organics is also good, so that there will be a high loss to the organic product stream. Finally, crown ethers are known chelating agents, so that there is a high probability of unacceptable loss of expensive hydrogenation catalyst metal, due to complexation with the crown ether.

In the method of the invention, the molar ratio of phase transfer catalyst to nitrobenzene reactant is preferably from 0.05:1 to 1.2:1.

The term "strong base" is intended to mean a base that is capable of abstracting a proton from the nitrogen of an aniline or aniline derivative, and may include any base having a pK_{b} less than 9.4, which is the pK_{b} of aniline. Various aniline derivatives may have different pK_{b} values, but a pK_{b} of 9.4 is employed as a general guide. The base will preferably have a pK_{b} less than 7.4.

While aniline most effectively couples with nitrobenzene, certain aniline derivatives comprising amides such as formanilide, phenylurea and carbanilide as well as the thiocarbanilide are substituted to produce 4-ADPA intermediates.

The method of the invention includes the step wherein the 4-ADPA intermediates from step (b) are subjected to a hydrogenation reaction involving the use of a hydrogenation catalyst Details concerning choice of catalyst and other aspects of the hydrogenation reaction may be found in U.S. Patent No. 6,140,538.

Other means of reduction, that do not involve the direct use of hydrogen and are known to one skilled In the art, can also be used to reduce the 4-ADPA intermediates 4-ADPA.

The present invention further relates to a process for preparing alkylated derivatives of 4-aminodiphenylamines, in particular for preparing alkyl derivatives of 4-ADPA itself, which are useful for the protection of rubber products, in which process an aniline derivative and nitrobenzene are coupled and subsequently reduced according to the invention process, after which the 4-aminodiphenylamine so obtained is reductively alkylated to an alkylated derivative of the 4-aminodiphenylamine according to methods known to the person skilled in this technical field. Typically, the 4-ADPA and a suitable ketone, or aldehyde, are reacted in the presence of hydrogen and platinum-on-carbon as catalyst. Suitable ketones include methylisobutyl ketone, acetone, methylisoamyl ketone, and 2-octanone. See for example U.S. 4,463,191, and Banerjee et al, J. Chem. Soc. Chem. Comm. 18, 1275-1276 (1988). Suitable catalysts can be the same as, but not limited to, those described above for obtaining the 4-ADPA.

In a preferred embodiment of the invention, the reduction is conducted in the presence of water, e.g. water is added to the reaction mixture. The use of water is particularly advantageous when the suitable base, used during the reaction of the aniline derivative and the nitrobenzene is water-soluble. When the base is water-soluble, the amount of water added is preferably at least the amount needed to extract the base from the organic phase. Similarly, the addition of water is also preferred for reductive alkylation, if it is carried out in the presence of the suitable base, which is water-soluble.

The molar ratio of aniline derivative to nitrobenzene in the process according to the present invention is not particularly important, as the process will be effective with an excess of either.

Strong bases of the present invention include potassium hydroxide, sodium hydroxide, cesium hydroxide, rubidium hydroxide and potassium-t-butoxide. It is preferred that mole ratio of strong base to nitrobenzene is greater than 1:1. A particularly preferred mole ratio of strong base to nitrobenzene is 2:1 to 6:1.

A particularly preferred combination of strong base and phase transfer catalyst is potassium hydroxide and tetraalkylammonium halide. A preferred halide is chloride. The above reactions would be carried out in aqueous solution with a continuous distillation of aniline-water azeotrope.

The reactive contact of the process of the embodiment of the invention is carried out in the presence of an oxidant. The oxidant may be free oxygen, or comprise an oxidizing agent such as a peroxide, particularly hydrogen peroxide. Nitrobenzene may also function as an oxidizing agent.

In the process of the invention, the oxidant may advantageously need to be present only for part of the time during which the aniline and nitrobenzene react. Such partial oxidative conditions are particularly effective for improving selectivity. Another instance is when TMAH is used as a strong base that can also function as a phase transfer catalyst. Moreover, although it has not been demonstrated for the embodiment of the process of the invention, it is believed that partial oxidative conditions would also be effective for combinations of inorganic base and phase transfer catalyst that give low selectivity.

The reactive contact may be carried out at a temperature of from 20°C to 150°C. Other conditions for the reactive contact include pressures in the range of from 20 mbar to 20 barg. Reaction time is typically less than 3.5 hours. It is advantageous to agitate the reaction mixture during the entire reaction.

The reactions of step (b) of the embodiments of the present method may be carried out in the presence of not greater than 10:1 moles water to moles nitrobenzene. The amount of water does not include the water that hydrates with the reactants and/or with compounds formed in the process. When the mixture comprising a strong base and a phase transfer catalyst, is in aqueous solution, the reaction may be carried out with a continuous distillation of aniline-water azeotrope.

The embodiment of the invention may be carried out with the phase transfer catalyst being tetramethylammonium bromide and the strong base comprising one or more inorganic bases.

The aqueous phase may be reused to form a new reaction mixture. Fresh base and phase transfer catalyst or organic base and inorganic salt or metal organic salt are added to replace losses by decomposition, by-product formation and solubility in the separated organic phase. Excess Aniline derivative recovered by distillation from the reaction product mixture may be combined with make-up fresh aniline derivative for recycle to form a new reaction mixture. Recovery of excess nitrobenzene is preferably carried out prior to hydrogenation of the 4-ADPA intermediate by a separation step and the recovered nitrobenzene may be combined with make-up fresh nitrobenzene for use in the process, or hydrogenated to aniline.

The method of the present invention for the preparation of 4-aminodiphenylamines intermediates may be conducted as a batch process or may be performed continuously using means and equipment well known to the skilled person.

The reactive contact in step (a) in the embodiment of the method of the invention may occur in a suitable solvent system. A suitable solvent system comprises a polar aprotic solvent The polar aprotic solvent may be selected form the group consisting of, but not limited to, dimethyl sulfoxide, benzyl ether, 1-Methyl-2-pyrrolidinone and N,N-dimethylformamide.

The invention is illustrated by the following example.

In the example the charging of the reactor was performed in open air and the reactor was stoppered resulting in some free oxygen being present during the reaction. No attempt was made to remove water from the reaction mixture in the example.

### ANALYTICAL

Yields of individual components were determined by external standard HPLC. Approximately 0.6 grams of material to be analyzed is accurately weighed into a 50-mL volumetric flask and diluted with a buffer solution containing 39% v/v water, 36% v/v acetonitrile, 24% v/v methanol and 1% v/v pH 7 buffer. The solution is injected through a 10 µL loop onto a reversed phase Zorbax ODS HPLC column (250 x 4.6 mm) using a binary gradient pumping system and the following elution gradient at a constant flow rate of 1.5 mL/minute:

| Time, minutes | %A | %B |
|---|---|---|
| 0 | 100 | 0 |
| 25 | 25 | 75 |
| 35 | 0 | 100 |
| 37.5 | 0 | 100 |
| 38 | 100 | 0 |
| 40 | 100 | 0 |

Eluent A is 75% v/v water, 15% v/v acetonitrile and 10% v/v methanol. Eluent B is 60% v/v acetonitrile and 40% v/v methanol. Detection is UV at 254 nm.

Conversion for the example is calculated by sum addition of known components plus any unknown peaks (assigned an arbitrary mole weight value of 216, aniline + nitrobenzene) as analyzed.

Selectivity is defined by the formula: (p-NDPA Yield + 4-NDPA Yield)/(total yield). 4-NDPA is 4-nitrodiphenylamine and p-NDPA is 4-nitrosodiphenylamine. Total yield is the sum of the yield of all known and unknown peaks (assigned an arbitrary mole weight value of 216, aniline + nitrobenzene).

In the table: "An Recr" refers to compounds from which aniline may be easily recovered and is a sum total of *trans*-azobenzene and azoxybenzene; "Other" are aniline and nitrobenzene coupling by-products e.g. phenazine, N-oxy-phenazine, 2-NDPA, 4-phenazo-diphenylamine and any unknowns.

### EXPERIMENTAL

The example shows that nitrobenzene may be coupled with a variety of aniline derivatives to produce 4-ADPA intermediates.

A stoichiometric amount of substrate as listed in the Table below; nitrobenzene (99%, 3.08 grams, 24.8 mmoles), potassium hydroxide (86% ground powder, 9.77 grams, 150 mmoles), tetramethylammonium chloride (97%, 2.74 grams, 24.3 moles), and water (2.84 grams) were charged to a 50-mL round bottom flask equipped with a magnetic stirrer. The reaction was allowed to proceed for 2 hours at 80°C in an open flask. Contents were then sampled and analyzed by HPLC.

**Table**

| Yield, % | | | | | |
|---|---|---|---|---|---|
| | Conversion | *p*-NDPA | 4-NDPA | An Recr | Other |
| Aniline, 99%, 2.33 grams, 24.8 mmoles | 95.4% | 6.0 | 68.3 | 19.9 | 1.2 |
| Formanilide, 99%, 3.03 grams, 24.8 mmoles | 84.5% | 19.3 | 47.3 | 16.3 | 1.5 |
| Phenylurea, 97%, 3.40 grams, 24.2 mmoles | 96.2% | 19.2 | 38.8 | 13.5 | 24.8 |
| Carbanilide, 98%, 2.65 grams, 12.2 mmoles | 48.1% | 1.3 | 37.1 | 9.3 | 0.4 |
| Thiocarbanilide, 98%, 2.85 grams, 12.2 mmoles | 58.6% | 5.4 | 31.6 | 18.6 | 3.0 |
| Acetanilide, 97%, 3.38 grams, 24.3 mmoles | 8.5% | 0.3 | 2.7 | 3.6 | 1.9 |
| Benzamide, 99%, 3.03 grams, 24.8 mmoles | 49.4% | 0.0 | 1.0 | 15.1 | 33.3 |
| N-Methyl-Benzamide, 99+%, 3.38 grams, 25.0 mmoles | 8.2% | 0.0 | 0.0 | 0.0 | 8.2 |
| Benzanilide, 98%, 2.47 grams, 12.3 mmoles | 0.1% | 0.0 | 0.1 | 0.0 | 0.0 |

While aniline most effectively couples with nitrobenzene in a KOH-TMACI system, amides such as formanilide, phenylurea and carbanilide as well as the thiocarbanilide can be substituted to produce 4-ADPA intermediates.

## Claims

1. A method of producing 4-aminodiphenylamine comprising the steps of:
(a) bringing an aniline derivative selected from the group consisting of formanilide, phenylurea, carbanilide and thiocarbanilide, into reactive contact with nitrobenzene;
(b) obtaining a 4-aminodiphenylamine intermediate product by reacting the aniline derivative and nitrobenzene in a confined zone at a suitable time, pressure and temperature, in the presence of a mixture comprising a strong base having pK_{b} less than 9.4, an oxidant, and a phase transfer catalyst, wherein the phase transfer catalyst is selected from the group consisting of tetramethylammonium chloride, tetramethylammonium fluoride, tetramethylammonium hydroxide, bis-tetramethylammonium carbonate, tetramethylammonium formate, tetramethylammonium acetate, tetrabutylammonium hydrogensulfate, tetrabutylammonium sulfate, methyltributylammonium chloride, benzyltrimethylammonium hydroxide, tricaprylmethylammonium chloride, tetrabutylammonium chloride, tetramethylammonium nitrate, cetyltrimethylammonium chloride, and choline hydroxide;
(c) reducing the 4-aminodiphenylamine intermediate product of step (b) to produce 4-aminodiphenylamine; and
(d) optionally, wherein the 4-aminodiphenylamine produced is reductively alkylated to an alkylated derivative of the 4-aminodiphenylamine

2. The method of claim 1 wherein the molar ratio of phase transfer catalyst to nitrobenzene is from 0.05 : 1 to 1.2 : 1.

3. The method of claim 1 wherein said strong base is selected from the group consisting of potassium hydroxide, sodium hydroxide, cesium hydroxide, rubidium hydroxide, and potassium-t-butoxide.

4. The method of claim 1 wherein the molar ratio of strong base to nitrobenzene is greater than 1: 1.

5. The method of claim 1 wherein the molar ratio of strong base to nitrobenzene is greater than 2 : 1 to 6 : 1.

6. The method of claim 1 wherein said oxidant is free oxygen.

7. The method of claim 1 herein said reactive contact is carried out at a temperature of from 20 °C to 150 °C, a pressure in the range of from 20 mbar to 20 barg and a reaction time less than 3.5 hours.

8. The method of claim 1 wherein the reaction of step (b) is carried out in the presence of not greater than 10 : 1 moles water to moles nitrobenzene excluding water of hydration.

9. The method of claim 1 wherein said mixture comprising a strong base and a phase transfer catalyst is in aqueous solution and the reaction is carried out with a continuous distillation of aniline-water azeotrope.

10. The method of claim 1 wherein said phase transfer catalyst is tetramethylammonium chloride.

11. The method of claim 1 wherein the reaction mixture is agitated during the entire reaction.

12. The method of claim 1 wherein said reactive contact occurs in a solvent system.

13. The method of claim 12 wherein said solvent system comprises a polar aprotic solvent.

14. The method of claim 13 wherein said polar aprotic solvent is selected from the group consisting of dimethyl sulfoxide, benzyl ether, 1-methyl-2 pyrrolidinone, and N,N-dimethylformamide.

15. The method of claim 1 wherein the 4-aminodiphenylamine produced is reductively alkylated to an alkylated derivative of the 4-aminodiphenylamine.

## Patentansprüche

1. Ein Verfahren zur Herstellung von 4-Aminodiphenylamin umfassend die Schritte:
(a) Reaktives in Kontakt bringen eines Anilinderivats ausgewählt aus der Gruppe bestehend aus Formanilid, Phenylhamstoff, Carbanilid und Thiocarbanilid mit Nitrobenzol;
(b) Erhalten eines 4-Phenylamin-Zwischenprodukts durch Umsetzen des Anilinderivats und Nitrobenzols in einem begrenzten Bereich über einen geeigneten Zeitraum und bei geeignetem Druck und Temperatur in Gegenwart eines Gemischs umfassend eine starke Base mit einem pK_{b} Wert kleiner als 9,4, einem Oxidationsmittel und einem Phasentransferkatalysator, wobei der Phasentransferkatalysator ausgewählt ist aus der Gruppe bestehend aus Tetramethylammoniumchlorid, Tetramethylammoniumfluorid, Tetramethylammoniumhydroxid, bis-Tetramethylammoniumcarbonat, Tetramethylammoniumformiat, Tetramethylammoniumacetat, Tetrabutylammoniumhydrogensulfat, Tetrabutylammoniumsulfat, Methyltributylammoniumchlorid, Benzyltrimethylammoniumhydroxid, Tricaprylmethylammoniumchlorid, Tetrabutylammoniumchlorid, Tetramethylammoniumnitrat, Cetyltrimethylammoniumchlorid und Cholinhydroxid;
(c) Reduzieren des Intermediatprodukts des 4-Aminodiphenylamins des Schrittes (b) um 4-Aminodiphenylamin herzustellen; und
(d) optional, wobei das hergestellte 4-Aminodiphenylamin reduzierend zu einem alkylierten Derivat des 4-Aminodiphenylamins alkyliert wird.

2. Verfahren nach Anspruch 1, wobei das molare Verhältnis des Phasentransferkatalysators zu Nitrobenzol von 0,05:1 bis 1,2:1 ist.

3. Das Verfahren nach Anspruch 1, wobei die starke Base ausgewählt ist aus der Gruppe bestehend aus Kaliumhydroxid, Natriumhydroxid, Cäsiumhydroxid, Rubidiumhydroxid und Kalium-t-butanolat.

4. Das Verfahren nach Anspruch 1, wobei das molare Verhältnis von starker Base zu Nitrobenzol größer als 1:1 1 ist.

5. Das Verfahren nach Anspruch 1, wobei das molare Verhältnis von starker Base zu Nitrobenzol größer als 2:1 bis 6:1 ist.

6. Das Verfahren nach Anspruch 1, wobei das Oxidationsmittel freier Sauerstoff ist.

7. Das Verfahren nach Anspruch 1, wobei das reaktive in Kontakt bringen bei einer Temperatur von 20 °C bis 150 °C, einem Druck im Bereich von 20 mbar bis 20 barg und einer Reaktionszeit weniger als 3,5 Stunden durchgeführt wird.

8. Das Verfahren nach Anspruch 1, wobei die Reaktion von Schritt (b) in Gegenwart von nicht mehr als 10:1 mol Wasser zu mol Nitrobenzol durchgeführt wird, ausgenommen das Wasser der Hydratisierung.

9. Das Verfahren nach Anspruch 1, wobei das Gemisch umfassend eine starke Base und einen Phasentransferkatalysator in wässriger Phase ist und die Reaktion mit einer fortlaufenden Destillation des Anilin-Wasser-Azeotrops durchgeführt wird.

10. Das Verfahren nach Anspruch 1, wobei der Phasentransferkatalysator Tetramethylammoniumchlorid ist.

11. Das Verfahren nach Anspruch 1, wobei das Reaktionsgemisch während der gesamten Reaktion geschüttelt wird.

12. Das Verfahren nach Anspruch 1, wobei das reaktive in Kontakt bringen in einem Lösungsmittelsystem stattfindet.

13. Das Verfahren nach Anspruch 12, wobei das Lösungsmittelsystem ein polares aprotisches Lösungsmittel umfasst.

14. Das Verfahren nach Anspruch 13, wobei das polare aprotische Lösungsmittel ausgewählt ist aus der Gruppe bestehend aus Dimethylsulfoxid, Benzylether, 1-Methyl-2-Pyrrolidinon und N,N-Dimethylformamid.

15. Das Verfahren nach Anspruch 1, wobei das hergestellte 4-Aminodiphenylamin reduzierend zu einem alkylierten Derivat des 4-Aminodiphenylamins alkyliert wird.

## Revendications

1. Procédé de production de la 4-aminodiphénylamine comprenant les étapes de:
(a) mettre un dérivé de l'aniline choisi dans le groupe consistant en formanilide, phénylurée, carbanilide et thiocarbanilide, en contact réactif avec le nitrobenzène;
(b) obtenir un produit intermédiaire de 4-aminodiphénylamine par réaction du dérivé de l'aniline et du nitrobenzène dans une zone confinée à une durée, une pression et une température appropriés, en présence d'un mélange comprenant une base forte ayant un pK_{b} inférieur à 9,4, un oxydant et un catalyseur à transfert de phase, où le catalyseur à transfert de phase est choisi dans le groupe consistant en le chlorure de tétraméthylammonium, le fluorure de tétraméthylammonium, l'hydroxyde de tétraméthylammonium, le carbonate de bis-tétraméthylammonium, le formiate de tétraméthylammonium, l'acétate de tétraméthylammonium, l'hydrogénosulfate de tétrabutylammonium, le sulfate de tétrabutylammonium, le chlorure de méthyltributylammonium, l'hydroxyde de benzyltriméthylammonium, le chlorure de tricaprylméthylammonium, le chlorure de tétrabutylammonium, le nitrate de tétraméthylammonium, le chlorure de cétyltriméthylammonium et l'hydroxyde de choline;
(c) réduire le produit intermédiaire de 4-aminodiphénylamine de l'étape (b) pour produire la 4-aminodiphénylamine; et
(d) éventuellement, où la 4-aminodiphénylamine produite est alkylée par réduction en un dérivé alkylé de la 4-aminodiphénylamine.

2. Procédé selon la revendication 1 où le rapport molaire du catalyseur à transfert de phase au nitrobenzène est de 0,05 : 1 à 1,2 : 1.

3. Procédé selon la revendication 1 où ladite base forte est choisie dans le groupe consistant en l'hydroxyde de potassium, l'hydroxyde de sodium, l'hydroxyde de césium, l'hydroxyde de rubidium et le t-butylate de potassium.

4. Procédé selon la revendication 1 où le rapport molaire de la base forte au nitrobenzène est supérieur à 1: 1.

5. Procédé selon la revendication 1 où le rapport molaire de la base forte au nitrobenzène est supérieur à 2 : 1 à 6 : 1.

6. Procédé selon la revendication 1 où ledit oxydant est l'oxygène libre.

7. Procédé selon la revendication 1 où ledit contact réactif est conduit à une température de 20°C à 150°C, une pression dans la plage de 20 mbar à 20 barg et une durée de réaction inférieure à 3,5 heures.

8. Procédé selon la revendication 1 où la réaction de l'étape (b) est conduite en présence de pas plus de 10 : 1 moles d'eau à moles de nitrobenzène à l'exclusion de l'eau d'hydratation.

9. Procédé selon la revendication 1 où ledit mélange comprenant une base forte et un catalyseur à transfert de phase est en solution aqueuse et la réaction est conduite avec une distillation continue de l'azéotrope aniline-eau.

10. Procédé selon la revendication 1 où ledit catalyseur à transfert de phase est le chlorure de tétraméthylammonium.

11. Procédé selon la revendication 1 où le mélange réactionnel est agité pendant toute la réaction.

12. Procédé selon la revendication 1 où ledit contact réactif a lieu dans un système solvant.

13. Procédé selon la revendication 12 où ledit système solvant comprend un solvant aprotique polaire.

14. Procédé selon la revendication 13 où ledit solvant aprotique polaire est choisi dans le groupe consistant en le diméthylsulfoxyde, le benzyléther, la 1-méthyl-2 pyrrolidinone et le N,N-diméthylformamide.

15. Procédé selon la revendication 1 où la 4-aminodiphénylamine produite est alkylée par réduction en un dérivé alkylé de la 4-aminodiphénylamine.
